# FASCICULE DE BREVET EUROPEEN

(11) **EP 0 893 987 B1**
(45) Date de publication et mention de la délivrance du brevet: **15.01.2003**
(21) Numéro de dépôt: 98905481.2
(22) Date de dépôt: 03.02.1998
(51) Int. Cl.: A61K 7/42, A61K 7/48

(54) **COMPOSITION ANTISOLAIRE CONTENANT UN ORGANOPOLYSILOXANE ELASTOMERIQUE SOLIDE**
SONNENSCHUTZMITTEL, DAS EIN FESTES ELASTOMERES ORGANOPOLYSILOXAN ENTHÄLT
SUNSCREEN COMPOSITION COMPRISING A SOLID ELASTOMERIC ORGANOPOLYSILOXANE

(30) Priorité: 17.02.1997 FR 9701811
(43) Date de publication de la demande: 03.02.1999
(73) Titulaire: L'OREAL, 75008 Paris (FR)
(72) Inventeur: HANSENNE, Isabelle, F-75017 Paris (FR)
(74) Mandataire: Miszputen, Laurent
(86) Numéro de dépôt international: FR9800191
(87) Numéro de publication internationale: WO98035649

(56) Documents cités:
- EP-A- 0 295 886
- EP-A- 0 610 026
- EP-A- 0 709 080
- FR-A- 2 509 989
- US-A- 5 266 321
- CHEMICAL ABSTRACTS, vol. 126, no. 8, 24 février 1997 Columbus, Ohio, US; abstract no. 108664, KURODA, AKIHIRO ET AL: "Sunscreens containing metal oxides, polyoxyalkylene-polysiloxanes, and elastomers or resin waxes" XP002047963 & JP 08 295 620 A (KANEBO LTD, JAPAN) 12 novembre 1996
- CHEMICAL ABSTRACTS, vol. 126, no. 3, 20 janvier 1997 Columbus, Ohio, US; abstract no. 36869, AIZAWA, MASANORI ET AL: "Cosmetics containing organopolysiloxane elastomers and UV absorbents for prevention of sunburn" XP002047964 & JP 08 259 419 A (SHISEIDO CO LTD, JAPAN)

## Description

L'invention se rapporte à une composition antisolaire du type émulsion contenant un organopolysiloxane élastomérique solide, présentant un degré de protection élevé contre le rayonnement solaire. Elle se rapporte aussi à un procédé de protection de la peau et/ou des lèvres contre les rayonnements du soleil consistant à appliquer sur la peau et/ou les lèvres cette composition.

Connaissant, de plus en plus les méfaits des rayons solaires (qu'ils soient naturels ou artificiels) sur la peau et le comportement excessif des êtres humains qui souhaitent toujours avoir « bonne mine », les cométiciens cherchent à fabriquer des compositions solaires de plus en plus performantes, du point de vue de la protection solaire et de la rémanence ou résistance à l'eau de ces compositions. Cette rémanence permet notamment aux êtres humains de se baigner après s'être recouverts de produit solaire, tout en se protégeant efficacement des rayons solaires.

Pour obtenir une composition à haut degré de protection, généralement représenté par le SPF (indice de protection solaire), on utilise notamment des émulsions, en particulier huile-dans-eau, contenant un ou plusieurs filtres hydrophiles ou hydrosolubles associés à ou plusieurs filtres lipophiles ou liposolubles particuliers au nom de la demanderesse. Selon cette technique, les améliorations du SPF obtenues reposent sur l'association synergique de filtres hydrophiles et lipophiles spécifiques (comme l'acide benzène 1,4-di(3-méthylidène-10-camphosulfonique) et l'α-cyano-β, β-diphénylacrylate de 2-éthylhéxyle (cas de la demande EP-A-0 685 228)) au sein d'émulsions huile-dans-eau classiques, de sorte que ces améliorations ne peuvent pas être obtenues de façon générale, c'est-à-dire quels que soient les filtres hydrophiles et lipophiles utilisés.

Les filtres lipophiles présentent l'avantage de conférer à la composition de bonnes propriétés de rémanence à l'eau. Ainsi, plus la concentration en filtres lipophiles augmente plus les propriétés de rémanence à l'eau et le pouvoir filtrant augmente. Or l'emploi de ces filtres lipophiles à haute concentration présente l'inconvénient de conférer à la composition un aspect gras et brillant, souvent ressenti comme un inconvénient notamment par les personnes à peau grasse.

On connaît dans le document EP0610026 des compositions cosmétiques solaires sous forme d'émulsion comprenant une phase aqueuse, une phase grasse contenant un filtre UV liposoluble et comme agent filmogène un latex de polyorganosiloxane réticulé élastomère afin d'améliorer l'adhérence de la composition sur la peau . On connaît également dans le document FR 2509989 des compositions solaires contenant une phase huileuse pouvant contenir un filtre solaire liposoluble et une phase aqueuse contenant un filtre solaire hydrosoluble. On connaît également dans le document EP0709080 des compositions solaires contenant une phase aqueuse pouvant contenir un filtre solaire hydrosoluble et une phase huileuse contenant au moins un filtre solaire liposoluble UV-A du type dérivé de dibenzoylméthane associé à au moins un polymère filtre solaire UV-B du type silicone à fonction benzylidène malonate, ledit polymère filtre siliconé étant utilisé comme filtre solaire actif dans l'UV-B et pour photostabilser ledit dérivé de dibenzoylméthane.

Ainsi, il subsiste le besoin d'une composition antisolaire comprenant à la fois des filtres hydrophiles et des filtre lipophiles, et dont le SPF serait élevé et stable dans le temps, quels que soient les filtres hydrophiles et/ou lipophiles utilisés, tout en présentant un aspect non gras.

La composition de l'invention s'applique aussi bien sur la peau que sur les lèvres. Elle peut se présenter sous forme de crème de soin ou de traitement ainsi que sous forme de produit de maquillage.

De façon plus précise, l'invention a pour objet une composition solaire comprenant une phase aqueuse contenant au moins un filtre hydrosoluble, une phase grasse contenant au moins un filtre liposoluble et au moins un organopolysiloxane solide élastomérique servant de gélifiant, selon la revendication 1.

Par « élastomérique » on entend un matériau souple, déformable ayant des propriétés viscoélastiques et présentant notamment la consistance d'une éponge ou d'une sphère souple.

Les organopolysiloxanes élastomériques de la composition selon l'invention présentent un remarquable pouvoir gélifiant d'huile. Ils ne sont pas desséchants pour la peau et apportent de bonnes propriétés cosmétiques. Ces nouveaux élastomères conduisent à des compositions confortables à l'application, douces, non grasses et non collantes au toucher. Cette douceur est due notamment à la texture des organopolysiloxanes.

La composition de l'invention peut se présenter sous forme de pâte ou de crème. Elle peut être une émulsion huile-dans-eau ou eau-dans-huile.

Les organopolysiloxanes élastomériques de la composition selon l'invention sont en général partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel plus ou moins homogène, en présence de quantités de phase grasse plus élevées. La gélification de la phase grasse par ces élastomères peut être totale ou partielle.

Les élastomères de la composition de l'invention sont véhiculés généralement sous forme de gel constitué d'un organopolysiloxane élastomérique de structure tridimensionnelle, inclus dans au moins une huile hydrocarbonée et/ou une huile siliconée et/ou fluorée.

Les organopolysiloxanes élastomériques de la composition selon l'invention peuvent être choisis parmi les polymères réticulés décrits dans la demande EP-A-0295886. Selon cette demande, Ils sont obtenus par réaction d'addition et de réticulation, en présence d'un catalyseur du type platine, d'au moins :
- (a) un organopolysiloxane ayant au moins deux groupes alcényle inférieurs par molécule ; et
- (b) un organopolysiloxane ayant au moins deux atomes d'hydrogène liés à un atome de silicium par molécule.

Les organopolysiloxanes élastomériques de la composition selon l'invention peuvent aussi être choisis parmi ceux décrits dans le brevet US 5 266 321. Selon ce brevet, ils sont choisis notamment parmi :
- i) les organopolysiloxanes partiellement réticulés comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

Les organopolysiloxanes objet de l'invention sont par exemple ceux commercialisés sous les noms KSG6 de Shin-Etsu, Trefil E-505C ou Trefil E-506C de Dow-Coming, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric, On peut aussi utiliser un mélange de ces produits commerciaux.

De façon préférentielle, le ou les organopolysiloxanes sont présents, en matière active, à une concentration allant de 0,1 à 80 % du poids total de la composition et de préférence de 0,5 à 60 %.

Les compositions cosmétiques antisolaires selon l'invention peuvent contenir un ou plusieurs filtres solaires hydrophiles actifs dans l'UVA et/ou l'UVB (absorbeurs), associés à un ou plusieurs filtres solaires lipophiles actifs dans l'UVA et/ou l'UVB. Ces filtres complémentaires peuvent être notamment choisis parmi les dérivés cinnamiques, les dérivés salicyliques (filtres lipophiles), les dérivés du camphre, les dérivés sulfoniques de benzimidazole, les dérivés de triazine (filtres lipophiles), les dérivés de la benzophénone, les dérivés du dibenzoylméthane, les dérivés de β,β-diphénylacrylate, les dérivés de l'acide p-aminobenzoïque (filtres hydrophiles), les polymères filtres et silicones filtres lipophiles, décrits dans la demande WO-93/04665.

### a) Les filtres hydrophiles

Comme filtres hydrophiles utilisables dans l'invention on peut citer ceux décrit dans la demande EP-A-678292. Ces filtres hydrophiles sont ceux contenant au moins un radical acide carboxylique ou mieux sulfonique. Ce radical acide peut être sous forme libre ou sous forme neutralisée, partiellement ou totalement. Selon l'invention, il est bien entendu possible d'utiliser un ou plusieurs filtres hydrophiles à fonction acide.

Comme exemple de filtres acides contenant au moins un groupe SO₃H, on peut plus particulièrement citer les dérivés sulfoniques du 3-benzylidène 2-camphre et notamment ceux de formules (I), (II), (III), (IV), et (V) suivantes :

### Formule (I) :

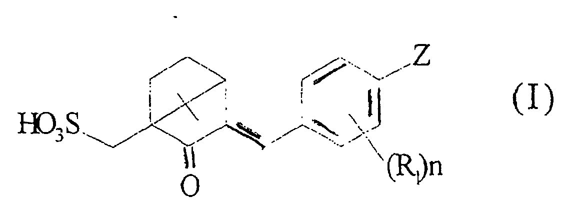

dans laquelle :
- Z désigne un groupement : 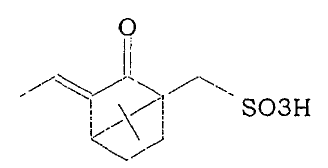
- n est égal à 0 ou est un nombre entier compris entre 1 et 4 (0 ≤ n ≤ 4),
- R₁ représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0, à savoir l'acide benzène 1,4 [di(3-méthylidènecampho 10-sulfonique)].

Ce filtre est un filtre à large bande capable d'absorber les rayons ultraviolets de longueur d'ondes comprises entre 280 nm et 400 nm, avec des maxima d'absorption compris entre 320 nm et 400 nm, en particulier aux alentours de 345 nm. Il est utilisé sous forme acide ou salifié par une base choisie parmi la triéthanolamine, la soude la potasse. En outre, il peut se présenter sous forme cis ou trans. Ce filtre est connu sous le nom commercial de *Mexoryl SX.*

### Formule (II) :

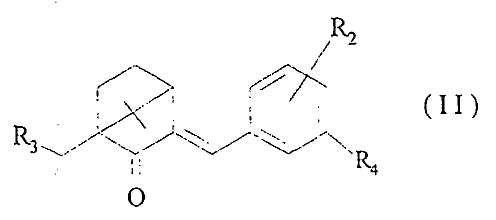

dans laquelle :
- R₂ désigne un atome d'hydrogène, un atome d'halogène, un radical alkyle contenant de 1 à 4 atomes de carbone environ ou un radical -SO₃H,
- R₃ et R₄ désignent un atome d'hydrogène ou un radical -SO₃H, l'un au moins des radicaux R₂, R3 ou R4 désignant le radical -SO₃H, R₂ et R₄ ne pouvant désigner simultanément un radical -SO3H.

On peut citer, comme exemples particuliers, les composés suivants de formule (II) dans laquelle :
- R₂ désigne le radical -SO₃H en position para du benzylidènecamphre et R₃ et R₄ désignent chacun un atome d'hydrogène, c'est-à-dire l'acide 4-(3-méthylidènecamphre) benzène sulfonique.
- R₂ et R₄ désignent chacun un atome d'hydrogène et R₃ désigne un radical -SO₃H, c'est-à-dire l'acide 3-benzylidène campho-10-sulfonique.
- R₂ désigne un radical méthyle en position para du benzylidènecamphre, R₄ un radical -SO₃H et R₃ un atome d'hydrogène, c'est-à-dire l'acide 2-méthyl 5-(3-méthylidènecamphre) benzène sulfonique.
- R₂ désigne un atome de chlore en position para du benzylidènecamphre, R₄ un radical -SO₃H et R₃ un atome d'hydrogène, c'est-à-dire l'acide 2-chloro 5-(3-méthylidènecamphre) benzène sulfonique.
- R₂ désigne un radical méthyle en position para du benzylidènecamphre, R₄ désigne un atome d'hydrogène et R₃ désigne un radical -SO₃H, c'est-à-dire l'acide 3-(4-méthyl) benzylidène campho 10-sulfonique.

### Formule (III) :

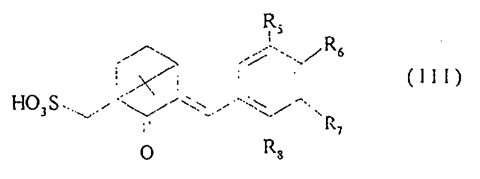

dans laquelle :
- R₅ et R₇ désignent un atome d'hydrogène, un radical hydroxyle, un radical alkyle ou alcoxy, linéaire ou ramifié, contenant de 1 à 8 atomes de carbone environ, l'un au moins des radicaux R₅ et R₇ représentant un radical hydroxyle, alkyle ou alcoxy,
- R₆ et R₈ désignent un atome d'hydrogène, un radical hydroxyle, l'un au moins des radicaux R₆ et R₈ désignant le radical hydroxyle, sous réserve que lorsque R₅ et R₈ désignent un atome d'hydrogène et que R₆ désigne un radical hydroxyle, R₇ ne désigne pas un radical alcoxy ou un atome d'hydrogène.

On peut citer, comme exemples particuliers, les composés suivants de formule (III) dans laquelle :
- R₅ est un radical méthyle, R₆ un atome d'hydrogène, R₇ un radical tertio-butyle, R₈ un radical hydroxyle, c'est-à-dire l'acide (3-t-butyl 2-hydroxy 5-méthyl) benzylidène campho-10-sulfonique.
- R₅ est un radical méthoxy, R₆ un atome d'hydrogène, R₇ un radical tertio-butyle, R₈ un radical hydroxyle, c'est-à-dire l'acide (3-t-butyl 2-hydroxy 5-méthoxy) benzylidène campho-10-sulfonique.
- R₅ et R₇ désignent chacun un radical tertiobutyle, R₆ un radical hydroxyle, R₈ un atome d'hydrogène, c'est-à-dire l'acide (3,5-diterbutyl 4-hydroxy) benzylidène campho-10-sulfonique.

### Formule (IV) :

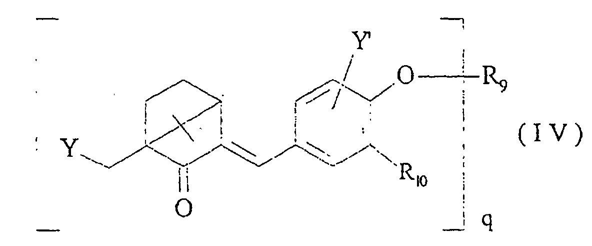

dans laquelle :
- R₉ désigne un atome d'hydrogène, un radical alkyle, linéaire ou ramifié, contenant de 1 à 18 atomes de carbone environ, un radical alcényle, linéaire ou ramifié, contenant de 3 à 18 atomes de carbone environ, un groupement 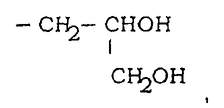 ou -(CH₂CH₂O)ₙ-H, ou -CH₂-CHOH-CH₃ ou encore un radical divalent : -(CH₂)ₘ- ou -CH₂-CHOH-CH₂-,
   n étant un nombre entier compris entre 1 et 6 (1 ≤ n ≤ 6) et m un nombre entier compris entre 1 et 10 (1 ≤ m ≤ 10),
- R₁₀ désigne un atome d'hydrogène, un radical alcoxy contenant de 1 à 4 atomes de carbone environ ou un radical divalent -O- relié au radical R₉ lorsque celui-ci est divalent lui aussi,
- q désigne un nombre entier égal à 1 ou 2, étant entendu que si q est égal à 2, R₉ doit désigner un radical divalent,
- Y et Y' désignent un atome d'hydrogène ou un radical -SO₃H, au moins un de ces radicaux Y ou Y' étant différent de l'hydrogène.

On peut citer, comme exemples particuliers, les composés suivants de formule (IV) dans laquelle :
- q est égal à 1, Y et R₁₀ désignent chacun un atome d'hydrogène, R₉ désigne un radical méthyle, Y' en position 3 désigne un radical -SO₃H, c'est-à-dire l'acide 2-méthoxy 5-(3-méthylidènecamphre) benzène sulfonique.
- q est égal à 1, Y désigne un radical -SO₃H, Y' un atome d'hydrogène, R₁₀ un radical divalent -O- relié à R₉ désignant un radical méthylène, c'est-à-dire l'acide 3-(4,5-méthylènedioxy) benzylidène campho-10-sulfonique.
- q est égal à 1, Y désigne un radical -SO₃H, Y' et R₁₀ désignent tous deux un atome d'hydrogène, R₉ désigne un radical méthyle, c'est-à-dire l'acide 3-(4-méthoxy) benzylidène campho-10-sulfonique.
- q est égal à 1, Y désigne un radical -SO₃H, Y' un atome d'hydrogène, R₉ désigne un radical méthyle, R₁₀ désigne un radical méthoxy, c'est-à-dire l'acide 3-(4,5-diméthoxy) benzylidène campho-10-sulfonique.
- q est égal à 1, Y désigne un radical -SO₃H, Y' et R₁₀ désignent tous deux un atome d'hydrogène, et R₉ un radical n butyle, c'est-à-dire l'acide 3-(4-n.butoxy) benzylidène campho-10-sulfonique.
- q est égal à 1, Y désigne un radical -SO₃H, Y' un atome d'hydrogène, R₉ désigne un radical n butyle, R₁₀ un radical méthoxy, c'est-à-dire l'acide 3-(4-n:butoxy 5-méthoxy) benzylidène campho-10-sulfonique.

### Formule (V) :

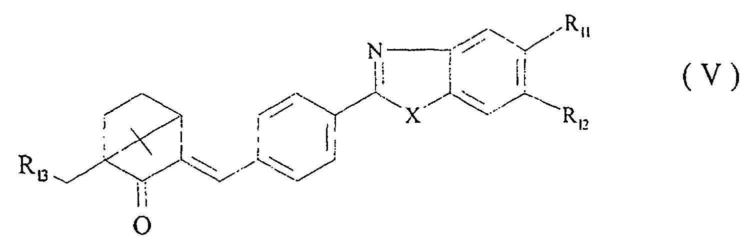

dans laquelle :
- R₁₁ désigne un atome d'hydrogène, un radical alkyle ou alcoxy, linéaire ou ramifié, contenant de 1 à 6 atomes de carbone environ ou un radical -SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H, l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer, comme exemple particulier de formule (V), le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de structures (I), (II), (III), (IV), (V) ci-dessus sont respectivement décrits dans le brevet US 4 585 597 et les demandes de brevets FR 2 236 515, 2 282 426, 2 645 148, 2 430 938 et 2 592 380.

Le filtre à groupement sulfonique peut également être un dérivé sulfonique de la benzophénone de formule (VI) suivante : 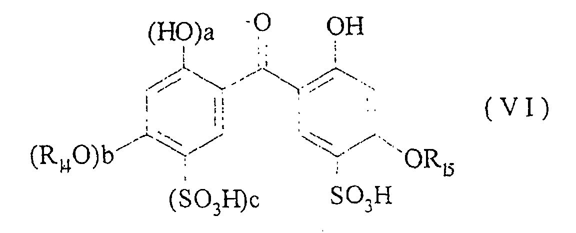 dans laquelle :
- R₁₄ et R₁₅, identiques ou différents, désignent soit un atome d'hydrogène soit un radical alkyle, linéaire ou ramifié, contenant de 1 à 8 atomes de carbone environ,
- a, b et c , identiques ou différents, sont des nombres égaux à 0 ou 1.

On peut citer comme exemple particulier de composé de formule (VI) : l'acide 2-hydroxy 4-méthoxybenzophénone 5-sulfonique (composé de formule (VI) dans laquelle a, b, et c sont égaux à zéro, et R₁₅ désigne un radical méthyle).

Le filtre à groupement sulfonique peut encore être un dérivé sulfonique de formule (VII) suivante : 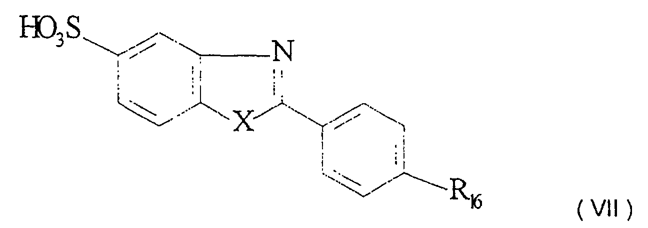 dans laquelle :
- X désigne un atome d'oxygène ou un radical -NH-,
- R₁₆ désigne un atome d'hydrogène, un radical alkyle ou alcoxy, linéaire ou ramifié, contenant de 1 à 8 atomes de carbone environ ou un groupement de formule (VIII) : 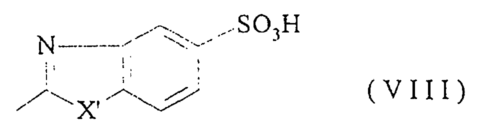
dans laquelle X' représente un atome d'oxygène ou un radical -NH-.

On peut citer, comme exemples particuliers, les composés suivants de formule (VII) dans laquelle :
- X désigne le radical -NH- et R₁₆ désigne un atome d'hydrogène : l'acide 2-phénylbenzimidazole 5-sulfonique. Ce filtre a excellent pouvoir photoprotecteur dans le domaine des rayonnements UV-B et est vendu sous la dénomination commerciale «EUSOLEX 232» par la société MERCK.
- X désigne le radical -NH-, R₁₆ désigne le groupement de formule (VIII) dans lequel X' désigne le radical -NH- : l'acide benzène 1,4 -di(benzimidazol -2 yl-5-sulfonique).
- X désigne un atome d'oxygène, R₁₆ désigne le groupement de formule (VIII) dans lequel X' désigne un atome d'oxygène : l'acide benzène 1,4-di (benzoxazol -2 yl -5-sulfonique).

Les composés de formule (VI) et (VII) sont des composés connus pouvant être préparés selon des méthodes classiques décrites dans l'art antérieur.

Le ou les filtres hydrophiles peuvent être présents dans la composition finale selon l'invention à une teneur pouvant varier de 0,1 à 20 %, de préférence de 0,2 à 10 %, en poids, par rapport au poids total de la composition.

### b) Les filtres lipophiles

Comme filtres lipophiles utilisables dans l'invention, on peut citer avantageusement la famille de filtres dérivés du dibenzoylméthane et plus spécialement le 4-tert.-butyl-4'-méthoxy dibenzoylméthane, qui présentent en effet un fort pouvoir d'absorption intrinsèque. Ces dérivés du dibenzoyl-méthane, qui sont des produits bien connus en soi à titre de filtres actifs dans l'UV-A, sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoylméthane est par ailleurs actuellement proposé à la vente sous la dénomination commerciale de "PARSOL 1789" par la Société GIVAUDAN.

Le 4-(ter.-butyl) 4'-méthoxy dibenzoylméthane présente la formule développée suivante :

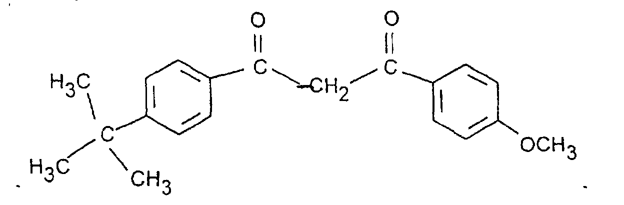

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

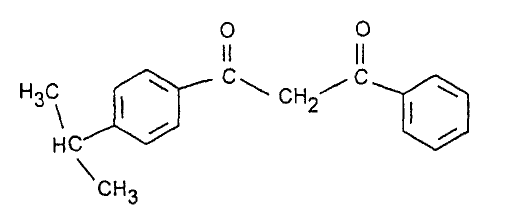

De même, l'α-cyano-β, β-diphénylacrylate de 2-éthylhéxyle, encore appelé octocrylène, est un filtre lipophile liquide déjà connu pour son activité dans l'UV-B. Il s'agit d'un produit qui est disponible commercialement, et est vendu notamment sous la dénomination de "UVINUL N 539" par la Société BASF. Il répond à la formule suivante : 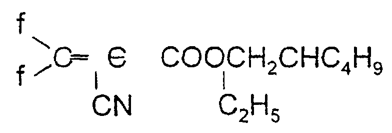 dans laquelle φ désigne un radical phényle.

Comme autre filtre lipophile (ou liposoluble) utilisable dans l'invention on peut encore citer le p-méthyibenzylidène camphre connu comme absorbant dans l'UV-B et vendu notamment sous la dénomination commerciale «EUSOLEX 6300» par la société Merck.

Le ou les filtres lipophiles peuvent être présents dans la composition selon l'invention à une teneur pouvant varier de 0,5 à 30%, de préférence de 0,5 à 20%, du poids total de la composition.

D'autres exemples de filtres organiques lipophiles ou hydrophiles sont donnés notamment dans la demande de brevet EP-A0487404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demande de brevets EP-A-0518772 etEP-A-0518773.

Les nanopigments peuvent être présents dans la composition selon l'invention à une teneur pouvant varier de 0,1 à 20%, de préférence de 0,2 à 10%, en poids, par rapport au poids total de la composition.

La phase grasse de la composition selon l'invention peut contenir une ou plusieurs huiles (liquides à température) et éventuellement une ou plusieurs cires (solides à température ambiante). Ces huiles et cires sont celles classiquement utilisées dans les domaines concernés.

Comme huiles utilisables dans l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides capryliquelcaprique comme ceux vendus par la société Stearineries Dubois ou ceux vendus sous les dénominations Miglyol 810, 812 et 818 par la société Dynamit Nobel ;
- les huiles de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine non volatiles et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'actyl dodécanol ou l'alcool oléique ;
- les huiles fluorées partiellement hydrocarbonées et/ou siliconées comme celle décrites dans le document JP-A-2-295912 ;
- les huiles siliconées comme les polyméthylsiloxanes volatiles ou non à température ambinate, linéaires ou cycliques, liquides ou pâteux à température ambiante, les phényl diméthicones, les phényl triméthicones et les polyméthylphénylsiloxanes ;
- leurs mélanges.

Les huiles représentent de 1 à 50% du poids total de la composition, de préférence entre 1 et 35% et sont choisies en fonction de leur compatibilité avec les organopolysiloxanes élastomériques.

Les cires peuvent être des cires hydrocrbonées, siliconées et/ou fluorées.

La composition de l'invention peut comprendre, en outre, tout additif usuellement utilisé dans le domaine concerné, des colorants hydrosolubles ou liposolubles, des antioxydants, des huiles essentielles, des conservateurs, des neutralisants, des électrolytes, des gélifiants de phase aqueuse ou grasse, des polymères liposolubles, des actifs cosmétiques ou dermatologiques comme par exemple des émollients, des hydratants, des vitamines, des actifs antirides, des acides gras essentiels, des charges et/ou des pigments et/ou des nacres colorés. Ces additifs peuvent être présents dans la composition à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition peut se présenter sous forme colorée ou non selon qu'elle constitue un produit de soin ou de maquillage.

Bien entendu la composition de l'invention doit être cosmétiquement ou dermatologiquement acceptable, à savoir non toxique et susceptible d'être appliqué sur la peau ou les lèvres d'êtres humains.

La composition de l'invention peut comprendre une phase particulaire, généralement présente à raison de 0 à 35% du poids total de la composition, de préférence de 5 à 25%, et qui peut comprendre des pigments et/ou des nacres et/ou des charges, colorés habituellement utilisés dans les compositions cosmétiques.

La composition selon l'invention peut être fabriquée par les procédés connus, généralement utilisés dans le domaine cosmétique ou dermatologique.
L'invention a encore pour objet un procédé cosmétique de soin ou de traitement de la peau ou des lèvres des êtres humains, comprenant l'application sur la peau ou les lèvres de la composition telle que définie ci-dessus.

L'invention est illustrée plus en détail dans les exemples suivants. Les pourcentages sont donnés en poids.

| **EXEMPLE 1 : Crème solaire** | |
|---|---|
| Glycérol | 6 |
| Triéthanolamine | 2,36 |
| Eau déminéralisée qsp | 100 |
| Conservateurs | 1,2 |
| Acide stéarique d'huile de palme | 2 |
| Arlacel 165 (Fer : ICI) | 1 |
| Heptanoate/Caprylate (67/30) de stéaryle | 2 |
| Bènzoate d'alcools C₁₂/C₁₅ | 9 |
| 4-tertiobutyl 4'-méthoxy dibenzoylméthane | 4 |
| Copolymère vinylpyrrolidone/eicosène | 1 |
| Hexadécylphosphate de potassium | 1 |
| 2,2,4,4,6,6,8 heptaméthylnonane | 3 |
| Oxyde de titane rutile (15nm) traité stéarate d'aluminium/albumine | 5 |
| Acide B-B'camphosulfonique [1-4divinylbenzène] en solution aqueuse à 33% | 9 |
| 2-cyano-3,3 diphénylacrylate de 2-éthyl hexyle Mélange poly diméthylsiloxane / poly diméthylsiloxane réticulé (76/24) | 10 |
| (KSG-16) | 2,5 |

Ce lait est une émulsion fluide huile-dans-eau douce, agréable au toucher, ni collante et ne brillant pas sur la peau, présentant des propriétés filtrantes rémanentes à l'eau et un indice de protection dans les UV très élevé de 105.

| **EXEMPLE 2 : Lait solaire** | |
|---|---|
| Glycérol | 4 |
| Triéthanolamine | 1,07 |
| Eau déminéralisée qsp | 100 |
| Conservateurs | 1 |
| Acide stéarique d'huile de palme | 2,2 |
| Arlacel 165 (Fer: ICI) | 1 |
| Heptanoate/caprylate (67/30) de stéaryle | 4 |
| Mélange polydiméthylsiloxane réticulé/poly méthylphényl siloxane (60/40) | 2,5 |
| 4-tertiobutyl 4'-méthoxy dibenzoylméthane | 2 |
| Silicone (Dow Corning 3225 C (Fer : Dow Corning)) | 1 |
| Mélange poly-diméthylsiloxane alpha-oméga dihydroxyle / cyclotétra et cyclopenta diméthylsiloxane (56/44) | 2,5 |
| Hexadécylphosphate de potassium | 1 |
| 2,2,4,4,6,6,8 heptaméthylnonane | 3 |
| Oxyde de titane rutile (15nm) traité stéarate d'aluminium/albumine | 5 |
| Acide B-B' camphosulfonique [1-4 divinylbenzène] en solution aqueuse à 33% | 3 |
| Cyclohexa diméthylsiloxane | 5 |
| 2-cyano-3,3-diphénylacrylate de 2-éthyl hexyle | 10 |

Ce lait du type huile-dans eau est non gras et non collant. Il présente un indice de protection élevé (SPF de 25) et une excellente rémanence à l'eau.

| **EXEMPLE 3 : Crème solaire** | |
|---|---|
| Silicone (DC 5200 formulation Aid Dow Corning) Poly méthyl lauryl / méthylsiloxane oxyéthytlène et oxypropylène Arlacel P 135 (ICI) | 2 |
| Di-polyhydroxy stéarate de polyéthylène glycol (30 DE) | 2 |
| Finsolv TN (stearinerie Dubois) | 6 |
| Benzoate d'alcools C₁₂ / C₁₅ Silicone (DC 246 fluid (Dow Corning) Cyclohexa diméthylsiloxane) | 10 |
| Conservateur | qs |
| TiO₂ T 805 (Degussa) | 3 |
| Uvinul N539 | 4 |
| Eusolex 6300 | 4 |
| Parsol 1789 | 2 |
| NaCI | 1 |
| Glycérine | 4 |
| Séquestrant | 0,3 |
| Mexoryl SX | 0,5 MA |
| Triéthanolamine | 0,26 |
| KSG 16 | 4 |
| Eau purifiée qsp | 100 |

Cette crème du type eau-dans-huile ne colle et ne brille pas après application sur la peau. Son indice de protection est de 35 et elle est résistante à l'eau.

**Analyse sensorielle comparative de 2 formules type eau-dans-huile, l'une contenant 4% de KSG-16, l'autre pas ;** les résultats sont donnés dans le tableau ci-après, l'évaluation cosmétique est notée de 0 à 5 (de mauvais à bon). Ces compositions correspondent à celle de l'exemple 3 avec ou sans KSG-16.

| | Témoin n° 1 | | Témoin n° 2 | | Témoin n° 3 | | Témoin n° 4 | | Témoin n° 5 | | Moyenne | |
|---|---|---|---|---|---|---|---|---|---|---|---|---|
| | Sans | 4% KSG -16 | Sans | 4% KSG-16 | Sans | 4% KSG-16 | Sans | 4% KSG 16 | Sans | 4% KSG 16 | Sans | 4% KSG 16 |
| Abs brillant | 2 | 4 | 2 | 4 | 3 | 5 | 2 | 5 | 2 | 4 | 2.20 | 4.40 |
| Abs gras | 3.5 | 3 | 3.5 | 3 | 2 | 3.5 | 3 | 5 | 2 | 4 | 2.80 | 3.70 |
| Abs collant | 2.5 | 2.5 | 3 | 3 | 2 | 4 | 4 | 5 | 3 | 4 | 2.90 | 3.70 |
| Douceur | 1 | 4 | 2 | 3 | 1 | 4 | 2 | 5 | 3 | 3 | 1.80 | 3.80 |

De ce tableau, il ressort clairement que l'introduction d'organopolysiloxane élastomérique permet d'augmenter les propriétés cosmétiques des compositions et notamment l'aspect non gras, non collant et la douceur.

Par ailleurs, la composition de l'exemple 1 avec du KSG présente un indice de protection de 105 alors que cette même composition sans KSG ne présente qu'un indice de protection de 80,3. Ainsi, en plus, de l'amélioration des propriétés cosmétiques des compositions, les organopolysiloxanes selon l'invention augmentent de façon notable l'indice de protection dans l'UV.

On obtient les mêmes types de résultats, à savoir amélioration des propriétés cosmétiques avec le lait de l'exemple 2 Par ailleurs, on note une augmentation de l'indice de protection (SPF), de 21,4 à 25,7.

## Revendications

1. Composition solaire contenant au moins une phase grasse contenant au moins un filtre solaire lipophile actif dans l'UVA et/ou l'UVB, au moins une phase aqueuse et au moins un organopolysiloxane solide élastomérique, **caractérisée par le fait qu'**elle contient au moins un filtre solaire hydrophile actif dans l'UVA et/ou l'UVB et que ledit organopolysiloxane, servant de gélifiant, est un organopolysiloxane solide élastomérique partiellement ou totalement réticulé de structure tridimensionnelle, choisi parmi :
(i) les organopolysiloxanes élastomériques réticulés obtenus par réaction d'addition et de réticulation, en présence d'un catalyseur du type platine d'au moins :
(a) un organopolysiloxane ayant au moins deux groupes alcényle inférieurs par molécule ;
(b) un organopolysiloxane ayant au moins deux atomes d'hydrogène liés à un atome de silicium par molécule ;
(ii) les organopolysiloxanes partiellement réticulés comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle, un aryle, un groupe aliphatique insaturé, le rapport en poids des motifs R₂SiO sur les motifs RSiO_{1,5} allant de 1/1 à 30/1 ;
(iii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

2. Composition selon la revendication 1, **caractérisée en ce que** la phase grasse contient une ou plusieurs huiles choisies parmi les huiles hydrocarbonées, silicosées et/ou fluorées.

3. Composition selon l'une quelconque des revendications précédentes, **caractérisée en ce que** la phase grasse représentent de 1 à 50 % du poids total de la composition.

4. Composition selon l'une quelconque des revendications précédentes, **caractérisée en ce que** le ou les filtres solaires hydrophiles représentent de 0,1 à 20 %, de préférence de 0,2 à 10 % du poids total de la composition

5. Composition selon l'une quelconque des revendications précédentes, **caractérisée en ce que** le ou les filtres solaire lipophiles sont présents de 0,5 à 30 %, de préférence de 0,5 à 20 % du poids total de la composition.

6. Composition selon l'une quelconque des revendications précédentes, **caractérisée en ce que** la composition contient, en outre, des nanopigments d'oxydes métalliques.

7. Composition selon la revendication précédente, **caractérisée en ce que** les nanopigments représentent de 0,1 à 20 %, de préférence de 0,2 à 10 %, du poids, total de la composition.

8. Composition selon l'une quelconque des revendications précédentes, **caractérisée en ce que** le filtre solaire hydrophile actif dans l'UVA et/ou l'UVB et le filtre solaire lipophile actif dans l'UVA et/ou l'UVB sont choisis dans le groupe constitué par :
les dérivés cinnamiques, les dérivés salicyliques, les dérivés du camphre, les dérivés sulfoniques de benzimidazole, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres lipophiles.

9. Composition selon l'une quelconque des revendications précédentes, **caractérisée en ce que** le ou les filtres solaires hydrophiles actifs dans l'UVA et/ou l'UVB sont choisis parmi les dérivés sulfoniques du 3-benzylidène 2-camphre, les dérivés sulfoniques de la benzophénone, les dérivés sulfoniques du benzimidazole, les dérivés de l'acide p-aminobenzoïque, l'acide benzène 1,4-di (benzoxazol -2 yl -5-sulfonique) et leurs mélanges.

10. Composition selon l'une quelconque des revendications précédentes, **caractérisée en ce que** le ou les filtres solaires lipophiles actifs dans l'UVA et/ou l'UVB sont choisis parmi le 4-(ter.-butyl) 4'-méthoxy dibenzoylméthane, l'isopropyl dibenzoylméthane, l'α-cyano-β, β-diphénylacrylate de 2-éthylhéxyle, le p-méthylbenzylidène camphre, les dérivés de triazine, les polymères filtres et silicones filtres lipophiles, les dérivés salicyliques et leurs mélanges.

11. Composition selon l'une quelconque des revendications précédentes, **caractérisée en ce qu'**elle contient, en outre, des charges et/ou des pigments et/ou des nacres, colorés.

12. Composition selon l'une quelconque des revendications précédentes, caractérisée en ce la composition contient, en outre, au moins un additif choisi parmi les antioxydants, les huiles essentielles, les conservateurs, les neutralisants, les gélifiants de phase grasse ou aqueuse, les électrolytes, les polymères liposolubles, les actifs cosmétiques ou dermatologiques.

13. Composition selon la revendication précédente, caractérisée en ce les actifs sont des émollients, des hydratants, des vitamines, des actifs antirides, des acides gras essentiels.

14. Composition selon l'une quelconque des revendications précédentes, caractérisée en ce la composition constitue une composition de soin, de traitement et/ou de maquillage.

## Patentansprüche

1. Sonnenschutzmittel, das mindestens eine Fettphase mit mindestens einem im UV-A- und/oder UV-B-Bereich wirksamen, lipophilen Filter, mindestens eine wäßrige Phase und mindestens ein festes elastomeres Organopolysiloxan enthält, **dadurch gekennzeichnet, daß** sie mindestens ein im UV-A- und/oder UV-B-Bereich wirksames, hydrophiles Filter enthält und dadurch, **daß** das Organopolysiloxan, das als Gelbildner dient, ein ganz oder teilweise vernetztes, festes, elastomeres Organopolysiloxan mit dreidimensionaler Struktur ist, das ausgewählt ist unter:
(i) den elastomeren vernetzten Organopolysiloxanen, die durch Addition und Vernetzung in Gegenwart eines Katalysators vom Platintyp zumindest aus den folgenden Verbindungen hergestellt werden:
- (a) einem Organopolysiloxan, das pro Molekül mindestens zwei niedere Alkenylgruppen aufweist, und
- (b) einem Organopolysiloxan, das pro Molekül mindestens zwei Wasserstoffatome aufweist, die an ein Siliciumatom gebunden sind,
(ii) den teilweise vernetzten Organopolysiloxanen, die Einheiten R₂SiO und RSiO_{1,5} und gegebenenfalls Einheiten R₃SiO_{0,5} und/oder SiO₂ aufweisen, worin die Gruppen R unabhängig voneinander Wasserstoff, eine Alkylgruppe, eine Arylgruppe oder eine ungesättigte aliphatische Gruppe bedeuten, wobei das Gewichtsverhältnis der Einheiten R₂SiO und der Einheiten RSiO_{1,5} im Bereich von 1/1 bis 30/1 liegt, und
(iii) den Organopolysiloxanen, die in einem Siliconöl unlöslich und quellfähig sind und die durch Addition eines Organohydrogenopolysiloxans (1) und eines Organopolysiloxans (2) mit ungesättigten aliphatischen Gruppen hergestellt werden, wobei die Wasserstoffmenge oder die Menge der ungesättigten aliphatischen Gruppen in (1) bzw. (2) im Bereich von 1 bis 20 Mol-%, wenn das Organopolysiloxan nicht cyclisch vorliegt, und im Bereich von 1 bis 50 Mol-% liegt, wenn das Organopolysiloxan cyclisch ist.

2. Zusammensetzung nach Anspruch 1, **dadurch gekennzeichnet, daß** die Fettphase ein oder mehrere Öle enthält, die unter den Kohlenwaserstoffölen, Siliconölen und/oder fluorierten Ölen ausgewählt sind.

3. Zusammensetzung nach einem der vorhergehenden Ansprüche, **dadurch gekennzeichnet, daß** die Fettphase 1 bis 50 % des Gesamtgewichts der Zusammensetzung ausmacht.

4. Zusammensetzung nach einem der vorhergehenden Ansprüche, **dadurch gekennzeichnet, daß** das oder die hydrophilen Filter 0,1 bis 20 % und vorzugsweise 0,2 bis 10 % des Gesamtgewichts der Zusammensetzung ausmachen.

5. Zusammensetzung nach einem der vorhergehenden Ansprüche, **dadurch gekennzeichnet, daß** das oder die lipophilen Filter 0,5 bis 30 % und vorzugsweise 0,5 bis 20 % des Gesamtgewichts der Zusammensetzung ausmachen.

6. Zusammensetzung nach einem der vorhergehenden Ansprüche, **dadurch gekennzeichnet, daß** die Zusammensetzung ferner Nanopigmente von Metalloxiden enthält.

7. Zusammensetzung nach dem vorhergehenden Anspruch, **dadurch gekennzeichnet, daß** die Nanopigmente 0,1 bis 20 % und vorzugsweise 0,2 bis 10 % des Gesamtgewichts der Zusammensetzung ausmachen.

8. Zusammensetzung nach einem der vorhergehenden Ansprüche, **dadurch gekennzeichnet, daß** das im UV-A-Bereich und/oder W-B-Bereich wirksame, hydrophile Filter und das im UV-A-Bereich und/oder UV-B-Bereich wirksame, lipophile Filter unter den folgenden Verbindungen ausgewählt sind: Zimtsäurederivaten, Salicylsäurederivaten, Campherderivaten, Sulfonsäurederivaten von Benzimidazol, Triazinderivaten, Benzophenonderivaten, Dibenzoylmethanderivaten, β,β-Diphenylacrylatderivaten, p-Aminobenzoesäurederivaten und lipophilen polymeren Filtern und Siliconfiltern.

9. Zusammensetzung nach einem der vorhergehenden Ansprüche, **dadurch gekennzeichnet, daß** das oder die im UV-A-Bereich und/oder UV-B-Bereich wirksamen, hydrophilen Filter unter den 3-Benzyliden-2-camphersulfonsäurederivaten, den Sulfonsäurederivaten von Benzophenon, den Sulfonsäurederivaten von Benzimidazol, p-Aminobenzoesäurederivaten, 1,4-Di(benzoxazol-2-yl-5-sulfonsäure) und deren Gemischen ausgewählt sind.

10. Zusammensetzung nach einem der vorhergehenden Ansprüche, **dadurch gekennzeichnet, daß** das oder die im UV-A-Bereich und/oder UV-B-Bereich wirksamen, lipophilen Filter unter 4-(t-Butyl)-4'methoxy-dibenzoylmethan, Isopropyldibenzoylmethan, 2-Ethylhexyl-α-cyano-β,β-diphenylacrylat, p-Methylbenzylidencampher, Triazinderivaten, lipophilen polymeren Filtern und Siliconfiltern, Salicylsäurederivaten und deren Gemischen ausgewählt sind.

11. Zusammensetzung nach einem der vorhergehenden Ansprüche, **dadurch gekennzeichnet, daß** sie ferner Füllstoffe und/oder Pigmente und/oder Perlglanzpigmente, die farbig sind, enthält.

12. Zusammensetzung nach einem der vorhergehenden Ansprüche, **dadurch gekennzeichnet, daß** die Zusammensetzung außerdem mindestens einen Zusatzstoff enthält, der unter den Antioxidantien, etherischen Ölen, Konservierungsmitteln, Neutralisationsmitteln, Gelbildnern für die wäßrige Phase und die Fettphase, Elektrolyten, fettlöslichen Polymeren oder kosmetischen oder dermatologischen Wirkstoffen ausgewählt ist.

13. Zusammensetzung nach dem vorhergehenden Anspruch, **dadurch gekennzeichnet, daß** der Wirkstoff unter den Emollientien, Hydratisierungsmitteln, Vitaminen, Wirkstoffen gegen Falten und essentiellen Fettsäuren ausgewählt ist.

14. Zusammensetzung nach einem der vorhergehenden Ansprüche, **dadurch gekennzeichnet, daß** die Zusammensetzung eine Zusammensetzung zur Pflege, zur Behandlung und/oder zum Schminken ist.

## Claims

1. Antisun composition containing at least one fatty phase containing at least one lipophilic sunscreen that is active in the UVA and/or UVB range, at least one aqueous phase and at least one elastomeric solid polyorganosiloxane, **characterized in that** it contains at least one hydrophilic sunscreen that is active in the UVA and/or UVB range and **in that** the said polyorganosiloxane, serving as gelling agent, is a partially or totally crosslinked, elastomeric solid polyorganosiloxane of three-dimensional structure, chosen from:
(i) the crosslinked elastomeric polyorganosiloxanes obtained by addition and crosslinking reaction, in the presence of a platinum-type catalyst, of at least:
(a) one polyorganosiloxane containing at least two lower alkenyl groups per molecule;
(b) one polyorganosiloxane containing at least two hydrogen atoms linked to a silicon atom per molecule;
(ii) the partially crosslinked polyorganosiloxanes comprising units R₂SiO and RSiO_{1.5} and optionally units R₃SiO_{0.5} and/or SiO₂ in which the radicals R, independently of each other, represent a hydrogen, an alkyl, an aryl or an unsaturated aliphatic group, the weight ratio of the units R₂SiO to the units RSiO_{1.5} ranging from 1/1 to 30/1;
(iii) organopolysiloxanes that are insoluble and swellable in a silicone oil, obtained by addition of an organohydrogenopolysiloxane (1) and an organopolysiloxane (2) containing unsaturated aliphatic groups such that the amount of hydrogen or of unsaturated aliphatic groups in (1) and (2), respectively, is between 1 and 20 mol% when the organopolysiloxane is non-cyclic and between 1 and 50 mol% when the organopolysiloxane is cyclic.

2. Composition according to Claim 1, **characterized in that** the fatty phase contains one or more oils chosen from hydrocarbon oils, silicone oils and/or fluoro oils.

3. Composition according to either of the preceding claims, **characterized in that** the fatty phase represents from 1% to 50% of the total weight of the composition.

4. Composition according to any one of the preceding claims, **characterized in that** the hydrophilic sunscreen (s) represent (s) from 0.1% to 20% and preferably from 0.2% to 10% of the total weight of the composition.

5. Composition according to any one of the preceding claims, **characterized in that** the lipophilic sunscreen(s) is (are) present in a proportion of from 0.5% to 30% and preferably from 0.5% to 20% of the total weight of the composition.

6. Composition according to any one of the preceding claims, **characterized in that** the composition also contains metal oxide nanopigments.

7. Composition according to the preceding claim, **characterized in that** the nanopigments represent from 0.1% to 20% and preferably from 0.2% to 10% of the total weight of the composition.

8. Composition according to any one of the preceding claims, **characterized in that** the hydrophilic sunscreen that is active in the UVA and/or UVB range and the lipophilic sunscreen that is active in the UVA and/or WB range are chosen from the group consisting of:
cinnamic derivatives, salicylic derivatives, camphor derivatives, sulphonic benzimidazole derivatives, triazine derivatives, benzophenone derivatives, dibenzoylmethane derivatives, β,β-diphenylacrylate derivatives, p-aminobenzoic acid derivatives, lipophilic screening polymers and lipophilic screening silicones.

9. Composition according to any one of the preceding claims, **characterized in that** the hydrophilic sunscreen(s) that is (are) active in the UVA and/or UVB range is (are) chosen from sulphonic 3-benzylidene-2-camphor derivatives, sulphonic benzophenone derivatives, sulphonic benzimidazole derivatives, p-aminobenzoic acid derivatives and benzene-1,4-bis(benzoxazol-2-yl-5-sulphonic) acid, and mixtures thereof.

10. Composition according to any one of the preceding claims, **characterized in that** the lipophilic sunscreen(s) that is (are) active in the UVA and/or UVB range is (are) chosen from 4-(tert-butyl)-4'-methoxydibenzoylmethane, isopropyldibenzoylmethane, 2-ethylhexyl α-cyano-β,β-diphenylacrylate, p-methylbenzylidenecamphor, triazine derivatives, lipophilic screening polymers and lipophilic screening silicones, and salicylic derivatives, and mixtures thereof.

11. Composition according to any one of the preceding claims, **characterized in that** it also contains coloured fillers and/or pigments and/or nacres.

12. Composition according to any one of the preceding claims, **characterized in that** the composition also contains at least one additive chosen from antioxidants, essential oils, preserving agents, neutralizers, fatty-phase or aqueous-phase gelling agents, electrolytes, liposoluble polymers and cosmetic or dermatological active agents.

13. Composition according to the preceding claim, **characterized in that** the active agents are emollients, moisturizers, vitamins, anti-wrinkle active agents and essential fatty acids.

14. Composition according to any one of the preceding claims, **characterized in that** the composition constitutes a care composition, a treatment composition and/or a makeup composition.
